# EUROPÄISCHE PATENTSCHRIFT

(11) **EP 3 374 763 B1**
(45) Veröffentlichungstag und Bekanntmachung des Hinweises auf die Patenterteilung: **17.03.2021**
(21) Anmeldenummer: 16794312.5
(22) Anmeldetag: 09.11.2016
(51) Int. Cl.: G01N 25/66

(54) **VORRICHTUNG UND VERFAHREN ZUR BESTIMMUNG DER FEUCHTIGKEIT EINER PROBE**
DEVICE AND METHOD FOR DETERMINING THE MOISTURE OF A SAMPLE
DISPOSITIF ET PROCÉDÉ POUR DÉTERMINER L'HUMIDITÉ D'UN ÉCHANTILLON

(30) Priorität: 09.11.2015 DE 102015119267
(43) Veröffentlichungstag der Anmeldung: 19.09.2018
(73) Patentinhaber: Brabender Messtechnik GmbH & Co. KG, 47055 Duisburg (DE)
(72) Erfinder: FREMUTH, Kay, 47239 Duisburg (DE); NABBEFELD, Tobias, 47665 Sonsbeck (DE)
(74) Vertreter: Michalski Hüttermann & Partner Patentanwälte mbB
(86) Internationale Anmeldenummer: PCT/EP2016/077169
(87) Internationale Veröffentlichungsnummer: WO 2017/081099

(56) Entgegenhaltungen:
- EP-A2- 0 520 472
- WO-A1-97/28434
- DD-A1- 155 115
- DE-A1- 2 247 569

## Beschreibung

Die Erfindung betrifft eine Vorrichtung zur Bestimmung der Feuchtigkeit einer ein Feststoffgemisch aufweisenden Probe, mit (i) mindestens einer Probenkammer zur Aufnahme der Probe, (ii) mindestens einem Sensor zur Messung einer Kenngröße eines die Probe umgebenden Gasgemischs und (iii) einer Ermittlungseinrichtung zur Ermittlung der Feuchtigkeit der Probe aus der mindestens einen Kenngröße. Die Erfindung betrifft weiterhin ein entsprechendes Verfahren zur Bestimmung der Feuchtigkeit einer ein Feststoffgemisch aufweisenden Probe.

Eine derartige Vorrichtung ist als Vorrichtung zum Bestimmen der Feuchtigkeit von Schüttgütern aus der DD 155 115 A1 bekannt. Bei dieser Vorrichtung ergibt sich ein Raum, beispielsweise in einer Förderschnecke zum Befördern des Schüttguts, in dem sich das Schüttgut selbst sowie ein Gasraum über diesem Schüttgut befinden. Die Vorrichtung weist einen Temperatursensor wie auch einen Taupunktfühler auf, die die Kenngrößen Temperatur und Taupunkt des in dem Gasraum befindlichen Gasgemisches messen. Die Ausgangssignale dieser Sensoren werden einer Ermittlungseinrichtung zur Ermittlung der Feuchte durch Wandlung und Verknüpfung der Ausgangssignale unter Berücksichtigung einer Taupunkt-Temperatur-Kurve zugeführt, die ihrerseits ein Ausgangssignal zur Anzeige oder für eine Regelung ausgibt. Die Genauigkeit der in dieser Druckschrift beschriebenen Bestimmung der Feuchtigkeit des Schüttguts ist relativ niedrig, nicht zuletzt, da nicht abgewartet wird, dass sich ein Gleichgewicht beim Wassergehalt zwischen Gas und Feststoffgemisch einstellt.

Um die Feuchtigkeit einer solchen ein Feststoffgemisch aufweisenden Probe deutlich genauer zu bestimmen, werden derzeit Verfahren und Vorrichtungen verwendet, die auf anderen Messprinzipien beruhen. Bei den bekannten Messprinzipien wird entweder ein Reagenz oder anderes Verbrauchsmaterial in Form von Chemikalien eingesetzt. Diese sind teilweise giftig, gesundheitsschädlich und/oder leicht entzündlich. Außerdem müssen sie nach einigen Messungen ersetzt werden. Das verbrauchte Material muss dann fachgerecht gesammelt und kostenintensiv entsorgt werden.

Die Druckschrift EP 0 520 472 A2 beschreibt eine Vorrichtung zur Bestimmung der Feuchtigkeit einer ein Feststoffgemisch aufweisenden Probe, mit mindestens einer Probenkammer zur Aufnahme der Probe, mindestens einem Sensor zur Messung einer Kenngröße eines die Probe umgebenden Gasgemischs, einer Ermittlungseinrichtung zur Ermittlung der Feuchtigkeit der Probe aus der mindestens einen Kenngröße und einer Messkammer zusätzlich zur Probenkammer.

Der Erfindung liegt die Aufgabe zugrunde, eine Vorrichtung und ein entsprechendes Verfahren zur Bestimmung der Feuchtigkeit einer Probe anzugeben, die eine zügige und genaue Bestimmung der Feuchte von Proben verbrauchsmaterialfrei ermöglichen.

Die Lösung der Aufgabe erfolgt erfindungsgemäß durch die Merkmale der unabhängigen Ansprüche. Vorteilhafte Ausgestaltungen der Erfindung sind in den Unteransprüchen angegeben.

Bei der erfindungsgemäßen Vorrichtung zur Bestimmung der Feuchtigkeit einer ein Feststoffgemisch aufweisenden Probe mit den im Oberbegriff des Anspruchs 1 genannten Merkmalen ist vorgesehen, dass die Vorrichtung eine evakuierbare Messkammer aufweist, die strömungstechnisch wahlweise von der mindestens einen Probenkammer abtrennbar und mit der Probenkammer verbindbar ist, wobei der mindestens eine Sensor eingerichtet ist, die Kenngröße des Gasgemisches in der Messkammer zu messen. Die Vorrichtung ist vorzugsweise eine Vorrichtung zur Bestimmung der Feuchte einer Schüttgutprobe oder einer sonstigen aus einem Feststoffgemisch bestehenden Probe.

Die Feuchtigkeit der Probe wird mittels der Vorrichtung wie folgt bestimmt: Zunächst wird die Probe in die Probenkammer verbracht und dort gegebenenfalls derart präpariert, dass sich in der Probenkammer das die Probe umgebende Gasgemisch in gewünschter Weise ausbildet. Dann wird die Probenkammer mit der zuvor evakuierten Messkammer strömungstechnisch verbunden, sodass ein Teil des die Probe umgebenden Gasgemisches aus der Probenkammer in die Messkammer strömt, wo die Messung der mindestens einen Kenngröße des Gasgemisches erfolgt. Anschließend wird mittels der Ermittlungseinrichtung aus der mindestens einen gemessenen Kenngröße die Feuchtigkeit der in der Messkammer befindlichen Probe ermittelt. Durch ein anschließendes strömungstechnisches Verbinden der evakuierten Messkammer mit der Probenkammer bzw. einer der Probenkammern kommt es zu einer Expansion des Gasgemischs aus der Probenkammer in die Messkammer, wobei das Größenverhältnis von Probenzu Messkammer so gewählt ist, dass sich das in die Messkammer gelangte Gasgemisch an einem für die genaue Messung benötigten Arbeitspunkt auf der Taupunktkurve befindet. Durch die Evakuierung ergibt sich eine Taupunkttemperatur T_{Taupunkt} von beispielsweise -20 °C. Das Volumen des Innenraums der Messkammer beträgt bevorzugt in etwa drei Liter. Die Probenkammer(n) hat/haben zumeist ein deutlich geringeres Innenvolumen.

Dabei weist die Vorrichtung zumindest eine Absperr-Armatur wie etwa ein Ventil oder einen Schieber auf, über die die mindestens eine Probenkammer wahlweise strömungstechnisch mit der Messkammer verbunden oder von der Messkammer getrennt werden kann.

Gemäß einer bevorzugten Ausgestaltung der Erfindung weist die Vorrichtung mindestens eine Heizung zum Heizen der Probe in der mindestens einen Probenkammer auf. Durch das Heizen wird die Probe auf eine gewünschte Temperatur gebracht und dadurch das die Probe umgebende Gasgemisch in wohldefinierter Weise ausgebildet. Die Heiztemperatur liegt dabei bevorzugt oberhalb von 100 °C, besonders bevorzugt im Bereich von 200 °C, also etwa 150 °C ≤ T_{H} ≤ 250 °C. Die Messkammer hat (zumindest zum Zeitpunkt der Messung) bevorzugt eine deutlich geringere Temperatur T_{M}, also T_{M} << T_{H}, vorzugsweise Raumtemperatur. Es sind in der Regel jedoch auch Messtemperaturen T_{M} von bis zu 60 °C möglich. Die Verwendung von Mess- und Probenkammer hat den Vorteil, dass der Sensor beziehungsweise die Sensoren nicht ausheizbar sein müssen, da sie ja in oder an der ungeheizten Messkammer angebracht sind.

Prinzipiell kann die Messkammer natürlich mit einer externen Vakuumpumpe evakuiert werden. Alternativ weist die Vorrichtung jedoch eine eigene Pumpe zum wahlweisen Evakuieren der Messkammer auf. Dabei ist es in der Regel völlig ausreichend, wenn die Pumpe die Messkammer bis in den Grobvakuumbereich, also in den Bereich einige hPa bzw. mbar Restgasdruck (beispielsweise 10 hPa) auszupumpen/leerzupumpen vermag. Die Pumpe wird bevorzugt über eine Absperr-Armatur, wie etwa ein Ventil oder einen Schieber wahlweise strömungstechnisch mit der Messkammer verbunden oder von der Messkammer getrennt.

Gemäß einer weiteren bevorzugten Ausgestaltung der Erfindung sind mehrere Probenkammern vorgesehen. Bei dieser Ausgestaltung können mehrere Proben parallel geheizt und die entsprechenden Kenngrößen mit relativ hoher Messrate, also hoher Repititionsrate der Messungen, gemessen werden.

Gemäß noch einer weiteren bevorzugten Ausgestaltung der Erfindung sind mehrere Sensoren vorgesehen. Diese Sensoren messen bevorzugt unterschiedliche Kenngrößen. Mit Vorteil ist einer der Sensoren ein Temperatursensor.

Generell gibt es eine Vielzahl von möglichen Sensoren für die Verwendung in der genannten Vorrichtung. An dieser Stelle seien beispielsweise ein coulometrischer P₂O₅-Sensor oder ein

Haarhygrometer genannt. Aus den Kenngrößen dieser Sensoren kann die Ermittlungseinrichtung anschließend die Feuchtigkeit der Probe ermitteln.

Gemäß einer bevorzugten Ausgestaltung der Erfindung ist jedoch vorgesehen, dass der Sensor oder zumindest einer der Sensoren ein Taupunktsensor ist. Es ergibt sich also eine Vorrichtung, dessen grundsätzliches Messprinzip gewisse Ähnlichkeit mit dem eingangs erwähnten Messprinzip der DD 155 115 A1 aufweist. Durch die erwähnten zusätzlichen Maßnahmen kann jedoch eine wesentlich höhere Genauigkeit bei der Bestimmung der Probenfeuchtigkeit erreicht werden.

Bei dieser Ausgestaltung der Erfindung ist insbesondere vorgesehen, dass der Taupunktsensor die Taupunkttemperatur mit Hilfe eines kapazitiven Sensorelementes bestimmt. Ein solches Sensorelement kann ein Sensorelement auf der Basis einer Metall-Keramik oder ein Polymer-Sensorelement sein. Die feuchteabhängige Kapazität und die Temperatur des Gases werden in einem Kalibrierablauf gemessen.

Alternativ ist vorgesehen, dass der Taupunktsensor einen Taupunktspiegel als Sensorelement aufweist. Dieser kann auch als Referenzinstrument für die Absolut-Feuchte eingesetzt werden.

Gemäß noch einer bevorzugten Ausgestaltung der Erfindung weist die Vorrichtung weiterhin eine mit dem mindestens einen Sensor signaltechnisch verbundene Mess-, Steuer- und/oder Regeleinrichtung auf, die auch die Ermittlungseinrichtung bildet. Bevorzugt ist die Mess-Steuer- und/oder Regeleinrichtung signaltechnisch auch mit der mindestens einen Heizung und/oder mit der Absperr-Armatur bzw. den Absperr-Armaturen verbunden. Mittels der Mess-Steuer- und/oder Regeleinrichtung wird der gesamte Prozess der Feuchtigkeitsbestimmung gesteuert beziehungsweise geregelt sowie am Ende die Feuchtigkeit der Probe aus der gemessenen Kenngröße oder den gemessenen Kenngrößen ermittelt.

Gemäß einer weiteren bevorzugten Ausgestaltung der Erfindung weist die Vorrichtung weiterhin mindestens ein Fluid-Kreislaufsystem auf, in dem die Messkammer strömungstechnisch verschaltet ist. Durch dieses Fluid-Kreislaufsystem beispielsweise kann die Restfeuchte des Messsystems gleichmäßig verteilt werden.

Mit Vorteil ist bei dieser Ausgestaltung vorgesehen, dass auch die mindestens eine Probenkammer in dem mindestens einen Fluid-Kreislaufsystem wahlweise verschaltbar oder permanent verschaltet ist. Durch diese Maßnahme kann das die Probe umgebende Gasgemisch rasch und gesteuert in die Messkammer eingeleitet werden.

Bei dem erfindungsgemäßen Verfahren zur Bestimmung der Feuchtigkeit einer ein Feststoffgemisch aufweisenden Probe mit den Schritten:
- Bereitstellen der Probe in einer Probenkammer,
- Messung einer Kenngröße eines die Probe umgebenden Gasgemischs und
- Ermittlung der Feuchtigkeit der Probe aus der mindestens einen Kenngröße,
   ist vorgesehen, dass die Messung der mindestens einen Kenngröße des Gasgemisches in einer strömungstechnisch mit der mindestens einen Probenkammer verbundenen Messkammer erfolgt, wohingegen sich das die Probe umgebende Gasgemisch zuvor in der strömungstechnisch von der Messkammer getrennten Probenkammer ausbildet. Um eine wohldefinierte Ausbildung des die Probe umgebenden Gasgemisches in der Probenkammer zu erreichen, wird die Probe in der Probenkammer vorzugsweise auf eine vorbestimmte Temperatur aufgeheizt.

Nachfolgend wird die Erfindung unter Bezugnahme auf die anliegenden Zeichnungen anhand bevorzugter Ausführungsbeispiele exemplarisch erläutert, wobei die nachfolgend dargestellten Merkmale sowohl jeweils einzeln als auch in Kombination einen Aspekt der Erfindung darstellen können. Dabei zeigt:
- Fig. 1: eine schematische Darstellung einer Vorrichtung zur Bestimmung der Feuchtigkeit einer ein Feststoffgemisch aufweisenden Probe gemäß einer bevorzugten Ausgestaltung der Erfindung,
- Fig. 2: die Vorrichtung zur Bestimmung der Feuchtigkeit einer ein Feststoffgemisch aufweisenden Probe gemäß einer weiteren bevorzugten Ausgestaltung der Erfindung bei einem Abpumpvorgang,
- Fig. 3: die in Fig. 2 gezeigte Vorrichtung bei einer Beruhigungsphase,
- Fig. 4: die in den Figuren 2 und 3 gezeigte Vorrichtung bei dem Messvorgang und
- Fig. 5: die in den Figuren 2 bis 4 gezeigte Vorrichtung bei einem Spülvorgang.

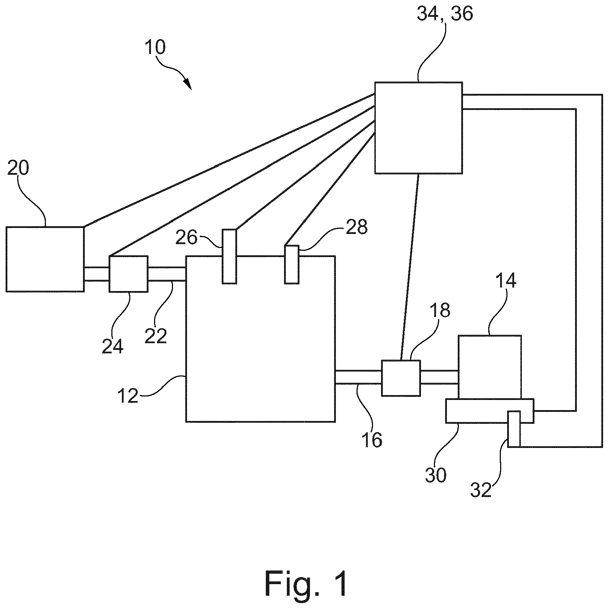

Die Fig. 1 zeigt eine Vorrichtung 10 zur Bestimmung der Feuchtigkeit einer ein Feststoffgemisch aufweisenden Probe (Probe nicht gezeigt). Die Vorrichtung weist als Hauptkomponenten eine Messkammer 12 sowie eine Probenkammer 14 zur Aufnahme der Probe auf. Generell kann die Vorrichtung 10 selbstverständlich auch mehrere solcher Probenkammern 14 aufweisen, da es sich hier jedoch nur um eine schematische Darstellung handelt, ist nur eine dieser Probenkammern 14 dargestellt. Das Volumen des Innenraums der Messkammer 12 beträgt bevorzugt in etwa 3 Liter. Die Probenkammer(n) 14 hat/haben zumeist ein deutlich geringeres Innenvolumen. Die Probenkammer 14 ist über ein als Verbindungsrohr ausgebildetes Fluid-Verbindungselement 16 mit der Messkammer 12 strömungstechnisch verbunden beziehungsweise in Fluid-Kommunikation. In dem Fluid-Verbindungselement 16 ist eine als Ventil ausgebildete Absperr-Armatur 18 verschaltet. Die Vorrichtung weist weiterhin eine Pumpe 20 zum wahlweisen Evakuieren der Messkammer 12 auf. Die Pumpe 20 ist ebenfalls über ein als Verbindungsrohr ausgebildetes Fluid-Verbindungselement 22 mit der Messkammer 12 strömungstechnisch verbunden beziehungsweise in Fluid-Kommunikation. Auch in diesem Fluid-Verbindungselement 22 ist eine als Ventil ausgebildete Absperr-Armatur 24 verschaltet. In beziehungsweise an der Messkammer 12 sind zwei Sensoren 26, 28 zur Messung von Kenngrößen eines sich im inneren der Messkammer befindlichen Gasgemisches befestigt. Der eine der Sensoren ist ein Taupunktsensor 26, der andere ein Temperatursensor 28. In beziehungsweise an der Probenkammer 14 ist eine Heizung 30 zum Heizen des Kammerinneren der Probenkammer 14, also insbesondere der dort befindlichen Probe, angebracht. An dieser Heizung (oder alternativ an der Probenkammer) ist auch ein Temperaturfühler 32 angebracht. Die Vorrichtung 10 weist weiterhin eine mit den Sensoren 26, 28, der mindestens einen Heizung 30 sowie dem Temperaturfühler 32, der Pumpe 20 sowie den Absperr-Armaturen 18, 24 signaltechnisch verbundene Mess-, Steuer- und/oder Regeleinrichtung 34 auf. Die genannten Komponenten 18, 20, 24, 30 sind von der Mess-, Steuer- und/oder Regeleinrichtung 34 ansteuerbar und die genannten Sensoren und der Messfühler 26, 28, 32 sind von der Mess- Steuer- und/oder Regeleinrichtung 34 auslesbar.

Es ergibt sich folgende Funktion:
Die zu messende Probe mit bekannter Dichte wird abgewogen und in die Probenkammer 14 verbracht. Die Armatur 18 zwischen Probenkammer 14 und Messkammer 12 ist geschlossen. Die Heizung 30 heizt die Probenkammer 14 und die darin befindliche Probe auf eine gewünschte Temperatur (beispielsweise etwa 200 °C) auf. Das Ventil 24 zwischen Pumpe 20 und Messkammer 12 ist geöffnet und die Messkammer 12 wird mittels der Pumpe 20 ausgepumpt. Nach ein paar Minuten (abhängig von der Größe der Messkammer 12 und Leistung der Pumpe 20) hat sich ein stabiler Unterdruck und damit auch ein entsprechender Taupunkt (also eine entsprechende Taupunkttemperatur) in der Messkammer 12 eingestellt und die Probenkammer 14 mit der darin befindlichen Probe hat eine konstante Temperatur für die Messung erreicht. Nun wird die Absperr-Armatur 24 zwischen Pumpe 20 und Messkammer 12 geschlossen und danach wird die Probenkammer 14 durch Öffnen der Absperr-Armatur 18 mit der leeren Messkammer 12 strömungstechnisch verbunden, sodass ein Teil des die Probe umgebenden Gasgemisches aus der Probenkammer 14 in die Messkammer 12 strömt, wo die Messung der Kenngröße dieses Gasgemisches erfolgt. Das Wasser aus der Probe geht durch die erhöhte Messtemperatur und den nun herabgesetzten Dampfdruck aus der Probe in die Gasphase über und verändert nun den Taupunkt in der Messkammer 12. Nach einiger Zeit ist der Taupunkt konstant.

Anschließend wird mittels einer von der Mess- Steuer- und/oder Regeleinrichtung 34 gebildeten Ermittlungseinrichtung 36 aus den gemessenen Kenngrößen die Feuchtigkeit der in der Messkammer 14 befindlichen Probe ermittelt. Mit der gemessenen Temperatur und dem Taupunkt lässt sich nämlich die vorhandene Menge Wasser pro Volumeneinheit berechnen. Da die Volumen im Messbereich und auch das Volumen der Probe (über Gewicht und Dichte) bekannt sind, kann somit die absolute Menge Wasser im System berechnet und mit dem ursprünglichen Gewicht der Probe ins Verhältnis gesetzt werden.

Die Verwendung von getrennter Mess- und Probenkammer 12, 14 hat also den Vorteil, dass der Sensor beziehungsweise die Sensoren 26, 28 nicht ausheizbar sein müssen, da sie ja in oder an der ungeheizten Messkammer 12 angebracht sind.

Jetzt kann das Fluid-System der Vorrichtung 10 belüftet werden und die nächste Probe gemessen werden. Da die Probenkammer 14 noch heiß ist, können weitere Probenkammern 14 über Verbindungelemente16 und Armaturen 18 mit der Messkammer 12 verbunden werden. Dadurch ist es möglich Messungen in kürzeren Zeitabständen anzusetzen.

Um die Genauigkeit der Feuchtigkeitsbestimmung zu erhöhen, wird vor der eigentlichen Messung der Taupunkt bestimmt und die Menge Wasser pro Volumeneinheit berechnet. Die absolute Menge Wasser, die sich in der Luft der Probenkammer 14 und dem angeschlossenen Verbindungselement 16 befindet, wird von der am Ende der Messung berechneten absoluten Menge Wasser im System abgezogen.

Mittels einer solchen Vorrichtung 10 zur Feuchtigkeitsbestimmung kann die Feuchtigkeit in einer Feststoffgemisch-Probe verbrauchsmaterialfrei bis hinab in den Bereich einiger ppm zuverlässig bestimmt werden. Eine Feuchtigkeitsbestimmung mit derartiger Genauigkeit ist beispielsweise bei der Verarbeitung von Feststoffgemisch-Proben wie etwa Kunststoff-Granulaten erwünscht.

Die gezeigte Vorrichtung 10 ist als mobiles Feuchtemessgerät, ja sogar als ein tragbares Feuchtemessgerät ausgebildet.

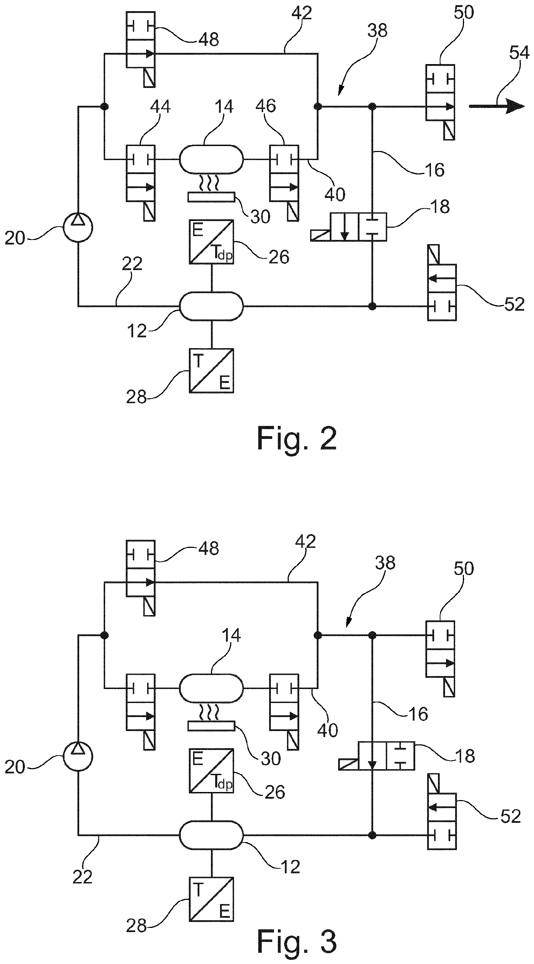

Die Figuren 2 bis 5 zeigen eine weitere Ausgestaltung der Vorrichtung 10 zur Bestimmung der Feuchtigkeit einer ein Feststoffgemisch aufweisenden Probe. Da die in den Figuren 2 bis 5 gezeigte Vorrichtung 10 im Wesentlichen der in Fig. 1 gezeigten Vorrichtung 10 entspricht, soll hier nur auf die Unterschiede eingegangen werden.

Die in den Figuren 2 bis 5 gezeigte Vorrichtung weist ein Fluid-Kreislaufsystem 38 auf, in dem die Messkammer 12 sowie die Pumpe 20 strömungstechnisch permanent verschaltet sind während die Probenkammer 14 wahlweise in diesem Fluid-Kreislaufsystem 38 verschaltbar ist. Dazu ist die Probenkammer 14 in einem Leitungsabschnitt 40 verschaltet, zu dem das Fluid-Kreislaufsystem 38 einen parallelgeschalteten Bypass 42 aufweist. Die Probenkammer 14 wird in dem Leitungsabschnitt 40 von zwei Absperr-Armaturen 44, 46 umgeben und auch der Bypass weist eine Absperr-Armatur 48 auf. Über diese Armaturen 44, 46, 48 kann wahlweise der Leitungsabschnitt 40 oder der Bypass 42 in das Kreislaufsystem 38 integriert werden. Dabei übernehmen diese Absperr-Armaturen 44, 46, 48 unter anderem auch die Funktion der aus der in Fig. 1 gezeigten Vorrichtung bekannten Absperr-Armatur 24.

Weiterhin weist die Vorrichtung 10 Be- und/oder Entlüftungsventile oder andere Armaturen 50, 52 auf, über die das Kreislauf-System 38 be- und/oder entlüftet werden kann. Sämtliche Armaturen 18, 44, 46, 48, 50, 52 können beispielsweise als Ventile ausgebildet sein und werden bevorzugt von der Mess- Steuer- und/oder Regeleinrichtung 34 angesteuert.

Bei der Bestimmung der Feuchtigkeit mittels dieser Ausführungsform der Vorrichtung 10 ergeben sich vier nacheinander ablaufende Phasen für die Messung der Kenngröße(n):
Fig. 2 zeigt die erste dieser Phasen, nämlich den Abpumpvorgang. Beim Abpumpvorgang wird die Messkammer 12 mittels der Pumpe 20 evakuiert. Die Probenkammer 14 ist durch die zwei Absperr-Armaturen 44, 46, die direkt vor und hinter der Probenkammer 14 positioniert sind, vom Rest des Kreislauf-Systems 38 getrennt. Dadurch kann mit dem Ausheizvorgang der Probe bereits in dieser Phase begonnen werden. Über den Bypass 42 kann das Gas aus der Messkammer 12 an der Probenkammer 14 vorbei abgepumpt werden und wird über die Armatur 50 in die Umgebung abgegeben (Pfeil 54). Der Abpumpvorgang wird beendet, sobald eine vorgegebene Taupunkttemperatur (typ. ≤ -20 °C) oder bei anderer Sensorwahl ein vorgegebener Unterdruck (typ. ca. 10 mbar absolut) erreicht wird.

Fig. 3 zeigt die zweite der Phasen, nämlich eine Beruhigungsphase. In der sogenannten Beruhigungsphase ist das Kreislauf-System 38 nach außen hin geschlossen. Auch die Probenkammer 14 ist weiterhin von der Messkammer 12 getrennt und wird weiterhin beheizt. Die Pumpe 20 lässt in dieser Phase das Restgas im System 38 zirkulieren. Diese Zirkulation des Restgases führt erstens dazu, dass die Restfeuchtigkeit, welche sich noch im System 38 befindet, gleichmäßig verteilt wird. Zweitens erreichen der relativ träge Taupunkttemperatursensor 26 und der relativ träge Temperatursensor 28 einen Gleichgewichtszustand und damit einen stabilen Wert. Dies ist wichtig, da die im System verbliebene Restfeuchtigkeit von der errechneten Feuchtigkeit der Probe am Ende des eigentlichen Messvorgangs abgezogen wird. Die Beruhigungsphase wird nach einer vorgegebenen Zeit (typ. ca. 2 Minuten) beendet.

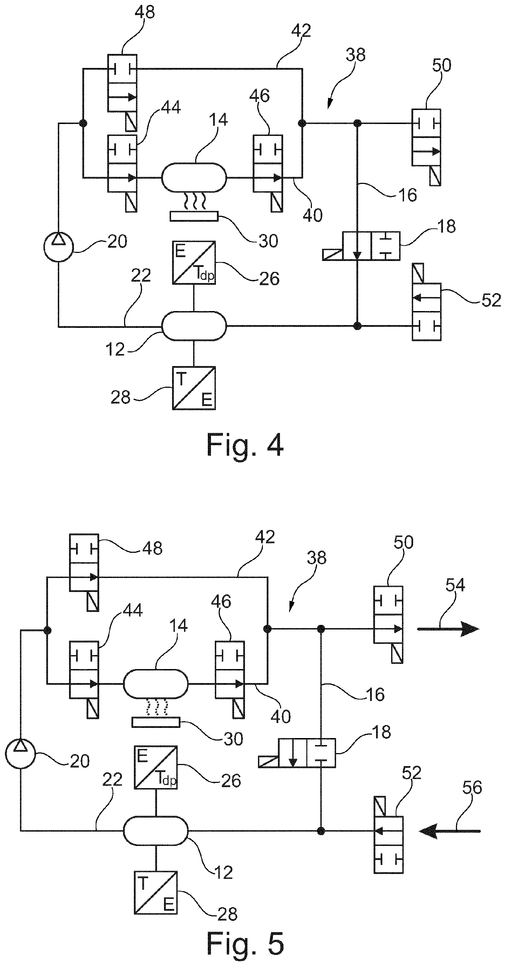

Fig. 4 zeigt die dritte der Phasen, nämlich die eigentliche Messphase. Zu Beginn der Messphase wird der Bypass 42 durch die Absperr-Armatur 48 abgesperrt. Die beiden Absperr-Armaturen 44, 46 vor und hinter der Probenkammer 14 werden geöffnet. Die Pumpe 20 sorgt dafür, dass das die Probe umgebende Gasgemisch aus der Probenkammer 14 im System 38 zirkuliert und so die Feuchtigkeit aus der Probe rasch und gleichmäßig im System 38 verteilt wird. Die Probe wird weiterhin ausgeheizt. Die Messphase bzw. der Messvorgang kann beendet werden, sobald der aus Taupunkttemperatur und Gastemperatur errechnete Wert für die im Gas enthaltene Wassermenge (Feuchtigkeit) konstant ist.

Die Fig. 5 zeigt schließlich die vierte der Phasen, nämlich einen Spülvorgang in Vorbereitung einer nächsten Messung. Beim Spülvorgang wird dafür gesorgt, dass die im System 38 enthaltene Feuchtigkeit das System 38 verlassen kann. Beide Armaturen 50, 52, die das Kreislauf-System 38 von der umgebenden Atmosphäre getrennt haben, werden nun geöffnet. Die Pumpe 20 sorgt für eine Strömung von umgebender Luft durch das komplette System 38, in welchem nun wieder Atmosphärendruck herrscht (Pfeile 54, 56). Sowohl die Strömung durch den Leitungsabschnitt 40 mit der Probenkammer 14 als auch durch den Bypass 42 sind vorgesehen. Die Probenkammer 14 wird nun nicht mehr geheizt. Die Luftströmung sorgt zudem für einen schnelleren Abkühlvorgang.

### Bezugszeichen

- 10: Vorrichtung
- 12: Messkammer
- 14: Probenkammer
- 16: Fluid-Verbindungselement
- 18: Absperr-Armatur
- 20: Pumpe
- 22: Fluid-Verbindungselement
- 24: Absperr-Armatur
- 26: Taupunktsensor
- 28: Temperatursensor
- 30: Heizung
- 32: Temperaturfühler
- 34: Mess- Steuer- und/oder Regeleinrichtung
- 36: Ermittlungseinrichtung
- 38: Fluid-Kreislaufsystem
- 40: Leitungsabschnitt
- 42: Bypass
- 44: Absperr-Armatur
- 46: Absperr-Armatur
- 48: Absperr-Armatur
- 50: Armatur
- 52: Armatur
- 54: Pfeil
- 56: Pfeil

## Patentansprüche

1. Vorrichtung (10) zur Bestimmung der Feuchtigkeit einer ein Feststoffgemisch aufweisenden Probe, mit
- mindestens einer Probenkammer (14) zur Aufnahme der Probe,
- mindestens einem Sensor (26, 28) zur Messung einer Kenngröße eines die Probe umgebenden Gasgemischs und
- einer Ermittlungseinrichtung (36) zur Ermittlung der Feuchtigkeit der Probe aus der mindestens einen Kenngröße,
**gekennzeichnet durch**
eine mittels einer externen Vakuumpumpe oder einer Pumpe (20) der Vorrichtung (10) in den Grobvakuumbereich evakuierbare Messkammer (12), die mittels einer Absperr-Armatur (18) der Vorrichtung (10) strömungstechnisch wahlweise von der mindestens einen Probenkammer (14) abtrennbar oder mit der Probenkammer (14) verbindbar ist, wobei der mindestens eine Sensor (26, 28) eingerichtet ist, die Kenngröße des Gasgemisches in der Messkammer (12) zu messen.

2. Vorrichtung nach Anspruch 1, **gekennzeichnet durch** mindestens eine Heizung (30) zum Heizen der Probe in der mindestens einen Probenkammer (14).

3. Vorrichtung nach Anspruch 1 oder 2, **dadurch gekennzeichnet, dass** mehrere Sensoren (26, 28) vorgesehen sind.

4. Vorrichtung nach einem der Ansprüche 1 bis 3, **dadurch gekennzeichnet, dass** der Sensor oder zumindest einer der Sensoren (26, 28) ein Taupunktsensor (26) ist.

5. Vorrichtung nach Anspruch 4, **dadurch gekennzeichnet, dass** der Taupunktsensor (26) den Wasserdampf-Partialdruck mit Hilfe eines kapazitiven Sensorelementes bestimmt.

6. Vorrichtung nach einem der Ansprüche 1 bis 5, **gekennzeichnet durch** eine mit dem mindestens einen Sensor (26, 28) signaltechnisch verbundene Mess-, Steuer- und/oder Regeleinrichtung (34), die die Ermittlungseinrichtung (36) bildet.

7. Vorrichtung nach einem der Ansprüche 1 bis 6, **gekennzeichnet durch** mindestens ein Fluid-Kreislaufsystem (38), in dem die Messkammer (12) strömungstechnisch verschaltet ist.

8. Vorrichtung nach Anspruch 7, **dadurch gekennzeichnet, dass** auch die mindestens eine Probenkammer (14) in dem mindestens einen Fluid-Kreislaufsystem (38) wahlweise verschaltbar oder permanent verschaltet ist.

9. Vorrichtung nach einem der Ansprüche 1 bis 8, **dadurch gekennzeichnet, dass** diese Vorrichtung (10) als mobiles Feuchtemessgerät, insbesondere als tragbares Feuchtemessgerät ausgebildet ist.

10. Verfahren zur Bestimmung der Feuchtigkeit einer ein Feststoffgemisch aufweisenden Probe, mit den folgenden Schritten:
- Bereitstellen der Probe in einer Probenkammer (14),
- Messung einer Kenngröße eines die Probe umgebenden Gasgemischs und
- Ermittlung der Feuchtigkeit der Probe aus der mindestens einen Kenngröße,
**dadurch gekennzeichnet, dass**
die Messung der mindestens einen Kenngröße des Gasgemisches in einer strömungstechnisch mit der mindestens einen Probenkammer (14) verbundenen Messkammer (12) erfolgt, wobei sich das die Probe umgebende Gasgemisch zuvor in der strömungstechnisch von der Messkammer (12) getrennten Probenkammer (14) ausbildet.

## Claims

1. An apparatus (10) for determining the moisture of a sample containing a solid mixture, with
- at least one sample chamber (14) for accommodating the sample,
- at least one sensor (26, 28) for measuring a characteristic variable of a gas mixture surrounding the sample, and
- a determination device (36) for determining the moisture content of the sample from the at least one characteristic variable,
**characterized by**
a measuring chamber (12) that can be evacuated into the rough vacuum range by means of an external vacuum pump or a pump (20) of the apparatus (10), which measuring chamber can be optionally fluidically separated from the at least one sample chamber (14) or connected to the sample chamber (14) by means of a separating fitting (18) of the apparatus (10), wherein the at least one sensor (26, 28) is set up to measure the characteristic variable of the gas mixture in the measuring chamber (12) .

2. The apparatus according to Claim 1, **characterized by** at least one heater (30) for heating the sample in the at least one sample chamber (14).

3. The apparatus according to Claim 1 or 2, **characterized in that** a plurality of sensors (26, 28) are provided.

4. The apparatus according to any one of Claims 1 to 3, **characterized in that** the sensor or at least one of the sensors (26, 28) is a dew point sensor (26).

5. The apparatus according to Claim 4, **characterized in that** the dew point sensor (26) determines the water vapour partial pressure by means of a capacitive sensor element.

6. The apparatus according to any one of Claims 1 to 5, **characterized by** a measuring, controlling and/or regulating device (34) which is connected to the at least one sensor (26, 28) by signalling means and forms the determination device (36).

7. The apparatus according to any one of Claims 1 to 6, **characterized by** at least one fluid circulation system (38) in which the measuring chamber (12) is fluidically connected.

8. The apparatus according to Claim 7, **characterized in that** the at least one sample chamber (14) can also be connected to the at least one fluid circulation system (38) or is optionally permanently connected therewith.

9. The apparatus according to any one of Claims 1 to 8, **characterized in that** said apparatus (10) is embodied as a mobile moisture meter, in particular as a portable moisture meter.

10. A method for determining the moisture of a sample containing a solid mixture, comprising the following steps:
- Providing the sample in a sample chamber (14),
- Measuring a characteristic variable of a gas mixture surrounding the sample and
- Determining the moisture of the sample from the at least one characteristic variable,
**characterized in that**
the measurement of the at least one characteristic variable of the gas mixture takes place in a measuring chamber (12) which is fluidically connected to the at least one sample chamber (14), wherein the gas mixture surrounding the sample is formed beforehand in the sample chamber (14) which is fluidically separated from the measuring chamber (12).

## Revendications

1. Appareil (10) pour déterminer l'humidité d'un échantillon comportant un mélange de substances solides, avec
- au moins une chambre à échantillon (14) pour recevoir l'échantillon,
- au moins un capteur (26, 28) pour mesurer une grandeur caractéristique d'un mélange gazeux entourant l'échantillon et
- un dispositif de détermination (36) pour déterminer l'humidité de l'échantillon à partir de l'au moins une grandeur caractéristique,
**caractérisé par**
une chambre de mesure (12) qui peut être vidée au moyen d'une pompe à vide externe ou d'une pompe (20) de l'appareil (10) pour obtenir un vide partiel et qui, du point de vue de la technique des fluides, peut être séparée de manière sélective de l'au moins une chambre à échantillon (14) ou reliée à la chambre à échantillon (14) au moyen d'un robinet d'arrêt (18) de l'appareil (10), l'au moins un capteur (26, 28) étant installé de manière à mesurer la grandeur caractéristique du mélange gazeux dans la chambre de mesure (12).

2. Appareil selon la revendication 1, **caractérisé par** au moins un chauffage (30) pour chauffer l'échantillon dans l'au moins une chambre à échantillon (14).

3. Appareil selon la revendication 1 ou 2, **caractérisé en ce que** plusieurs capteurs (26, 28) sont prévus.

4. Appareil selon l'une des revendications 1 à 3, **caractérisé en ce que** le capteur ou au moins l'un des capteurs (26, 28) est un capteur à condensation (26).

5. Appareil selon la revendication 4, **caractérisé en ce que** le capteur à condensation (26) détermine la pression partielle de vapeur d'eau à l'aide d'un élément capteur capacitif.

6. Appareil selon l'une des revendications 1 à 5, **caractérisé par** un dispositif de mesure, commande et/ou réglage (34) qui est relié du point de vue de la technique des signaux à l'au moins un capteur (26, 28) et qui forme le dispositif de détermination (36).

7. Appareil selon l'une des revendications 1 à 6, **caractérisé par** au moins un système de circulation de fluide (38) dans lequel la chambre de mesure (12) peut être intégrée du point de vue de la technique des fluides.

8. Appareil selon la revendication 7, **caractérisé en ce que** l'au moins une chambre à échantillon (14) peut aussi être intégrée de manière sélective ou est intégrée en permanence dans l'au moins un système de circulation de fluide (38).

9. Appareil selon l'une des revendications 1 à 8, **caractérisé en ce que** cet appareil (10) est réalisé sous forme d'hygromètre mobile, en particulier sous forme d'hygromètre portable.

10. Procédé de détermination de l'humidité d'un échantillon comportant un mélange de substances solides, avec les étapes suivantes
- placement de l'échantillon dans une chambre à échantillon (14),
- mesure d'une grandeur caractéristique d'un mélange gazeux entourant l'échantillon et
- détermination de l'humidité de l'échantillon à partir de l'au moins une grandeur caractéristique,
**caractérisé en ce que**
la mesure de l'au moins une grandeur caractéristique du mélange gazeux s'effectue dans une chambre de mesure (12) reliée du point de vue de la technique des fluides à l'au moins une chambre à échantillon (14), le mélange gazeux qui entoure l'échantillon se formant préalablement dans la chambre à échantillon (14) séparée de la chambre de mesure (12) du point de vue de la technique des fluides.
